(19) Europäisches Patentamt
European Patent Office
Office européen des brevets

(11) Veröffentlichungsnummer: **0 210 565**
**B1**

(12) **EUROPÄISCHE PATENTSCHRIFT**

(45) Veröffentlichungstag der Patentschrift: **24.10.90**

(51) Int. Cl.$^5$: **C 07 D 249/06** // C08K5/3472

(21) Anmeldenummer: **86109961.2**

(22) Anmeldetag: **21.07.86**

# (54) Stilbenverbindungen.

(30) Priorität: **01.08.85 DE 3527530**

(43) Veröffentlichungstag der Anmeldung:
**04.02.87 Patentblatt 87/06**

(45) Bekanntmachung des Hinweises auf die Patenterteilung:
**24.10.90 Patentblatt 90/43**

(84) Benannte Vertragsstaaten:
**CH DE FR GB LI**

(56) Entgegenhaltungen:
**US-A-3 761 470**
**US-A-3 928 329**
**US-A-3 965 094**
**US-A-3 994 834**
**US-A-4 366 189**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Seng, Florin, Dr.**
**Am Katterbach 46**
**D-5060 Bergisch-Gladbach 2 (DE)**
Erfinder: **Schellhammer, Carl-W., Dr.**
**Katharinental 26**
**D-5060 Bergisch-Gladbach 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 210 565 B1

**Beschreibung**

Gegenstand der Erfindung sind Stilbenverbindungen der Formel

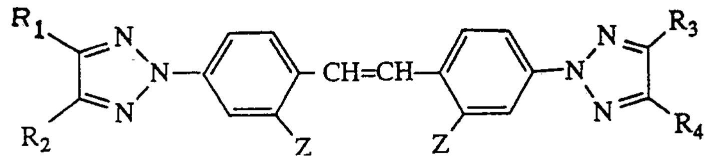

(I)

worin

$R_1$—$R_4$ unabhängig voneinander Wasserstoff oder $C_1$—$C_4$-Alkyl und

Z $C_1$—$C_4$-Alkoxy bedeuten.

Bevorzugter Alkoxyrest ist Methoxy.

Vorzugsweise stehen $R_1$ und $R_2$ bzw. $R_3$ und $R_4$ nicht gleichzeitig für Wasserstoff.

Man erhält die neuen Stilbenverbindungen dadurch, daß man in an sich bekannter Weise (vgl. z.B. DE 2 258 276) Aldehyde der Formel

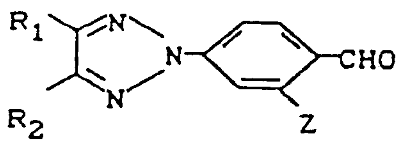

II

mit Phosphonsäureestern der Formel

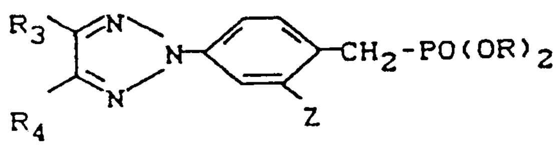

III

($R = C_1-C_4$-Alkyl)

kondensiert. Diese Reaktion erfolgt vorteilhafterwise in einem indifferenten Lösungsmittel in Gegenwart basischer Katalysatoren.

Während die Aldehyde der Formel II bekannt sind (vgl. DE 23 35 218), sind die Phosphonsäureester (III) bislang nicht beschrieben worden. Man erhält diese aber leicht nach folgendem Reaktionsschema:

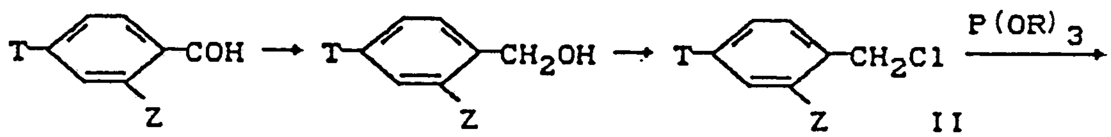


Die neuen Stilbenverbindungen eignen sich hervorragend zum Masse-Weißtönen von thermoplastischen Kunststoffen, wie z.B. PVC, Polyester, Polyethylen, Polypropylen, Polystyrol, Polycarbonat und Polymethacrylat.

Dabei wird die Stilbenverbindung gegebenenfalls mit dem Kunststoffgranulat trocken vermischt oder vermahlen und dieses Gemisch z.B. auf Mischwalzen oder in Schnecken plastifiziert und homogenisiert. Man kann den Weißtöner auch der schmelzflüssigen Masse zugeben und diese durch Rühren homogen verteilen. Des derart vorbehandelte Material wird dann wie üblich, z.B. durch Verspinnen zu Borsten, Fäden usw., oder durch Extrusion oder im Spritzgußverfahren zu Formteilen weiterverarbeitet.

Die auf diese Weise weißgetönten Kunststoffe zeichnen sich bei guten Allgemeinechtheiten durch einen hohen Weißgrad aus.

Aus US—A 3 761 470 sind ähnliche Stilbenverbindungen bekannt, die sich auch zum Weißtönen von Polymeren in der Masse eignen, aber sich in 3 Positionen von der Struktur der erfindungsgemäßen Weißtöner unterscheiden. Strukturell näher vergleichbar sind Stilbenverbindungen gemäß US—A 3 994 834, die lediglich anstelle der chaakteristischen Alkoxygruppe an den Benzolringen eine Sulfogruppe tragen.

Beispiel 1

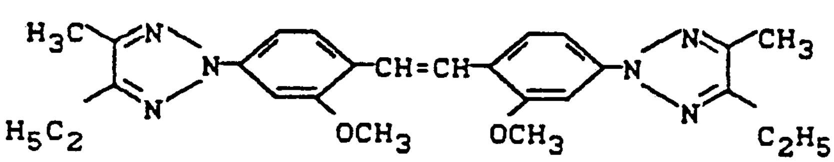

4,9 g (0,02 mol) 2-Methoxy-4-[4-methyl-5-ethyl-1,2,3-triazolyl(2)]-benzaldehyd werden zusammen mit 7,4 g (0,02 mol) 2-Methoxy-4-[4-methyl-5-ethyl-1,2,3-triazol-(2)]-benzyl-diethyl-phosphonat in 50 ml Dimethylformamid vorgelegt. Unter Stickstoff tropft man bei Raumtemperatur 4 ml (0,02 mol) einer 30 %igen Natriummethyllösung zu und rührt 2 Studen bei 60°C nach. Anschließend wird abgekühlt und abgesaugt. Man erhält 7,5 g 2,2'-Bis-methoxy-4,4'-bis-[4-methyl-5-ethyl-1,2,3-triazolyl(2)]stilben als gelbe Kristalle, die nach dem Umlösen aus Toluol/Tonsil bei 197 bis 199°C schmelzen $\lambda_{max}$ = 371,7 nm (DMF).

Beispiel 2

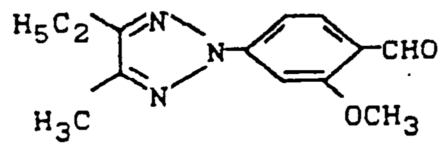

52,5 g (0,2 mol) 88 %iger 2-Hydroxy-4-[4-methyl-5-ethyl-1,2,3-triazolyl(2)]-benzaldehyd werden zusammen mit (27,6 g (0,2 mol) Kaliumcarbonat in 250 ml Aceton vorgelegt. Man erhitzt zum Sieden und tropft in der Siedehitze 27,7 g (0,22 mol) Dimethylsulfat zu. Man rührt noch 3,5 Stunden in der Siedehitze nach, kühlt dann ab und versetzt den Kolbeneinhalt mit 500 ml Wasser. Das ausgefallene weiße Produkt wird abgesaugt und getrocknet. Man erhält 45 g 2-Methoxy-4-[4-methyl-5-ethyl-1,2,3-triazolyl[2]-benzaldehyd vom Schmelzpunkt: 108 bis 111°C.

Beispiel 3

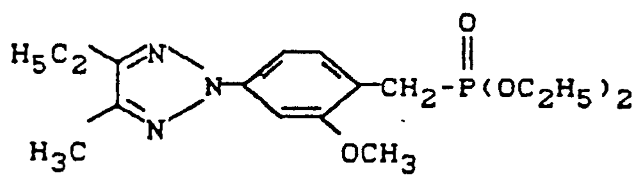

16,2 g (0,061 mol) 2-Methoxy-4-[4-methyl-5-ethyl-1,2,3-triazolyl(2)]-benzylchlorid und 51 g Triethylphosphit werden zum Sieden erhitzt. Nach dem Ende der Ethylchloridentwicklung (ca. 3 Stunden) wird das überschüssige Triethylphosphit bei einer Badtemperatur von 100°C im Wasserstrahlvakuum abdestilliert. Es hinterbleiben 24 g 2-Methoxy-4-[4-methyl-5-ethyl-1,2,3-triazolyl(2)]-benzyl-diethyl-phosphonat als farbloses Öl, das beim Abkühlen kristallin erstarrt. Fp: 41 bis 45°C.

Beispiel 4

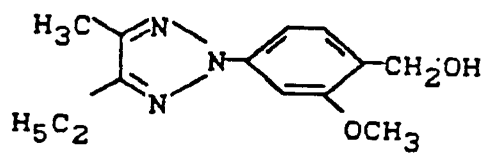

44,1 g (0,18 mol) 2-Methoxy-4-[4-methyl-5-ethyl-1,2,3-triazolyl(2)]-benzaldehyd werden in 250 ml Methanol gelöst und innerhalb von 2 Stunden bei 0 bis 5°C mit 4 g Nariumboronat versetzt. Man rührt 2 Stunden bei Raumtemperatur nach und säuert dann vorsichtig ($H_2$-Entwicklung!) mit Essigsäure an. Anschließend wird im Wasserstrahlvakuum einrotiert. Der hinterbleibende Rückstand von 2-Methoxy-4-[4-methyl-5-ethyl-1,2,3-triazolyl(2)]-benzylalkohol wird direkt in Beispiel 9 weiterverarbeitet.

Beispiel 5

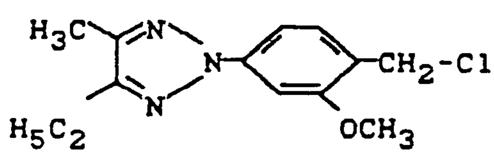

Der in Beispiel 4 erhaltene Rückstand wird in einer Mischung aus 200 ml 37 %iger Salzsäure und 200 ml Methylenchlorid 3 Stunden bei Raumtemperatur gerührt. Anschließend trennt man die organische Phase im Scheidetrichter ab und trocknet sie über Natriumsulfat. Das Natriumsulfat wird abfiltriert und das Filtrat im Wasserstrahlvakuum einrotiert. Den hinterbleibenden Rückstand kristallisiert man aus 260 ml Cyclohexan/Tonsil um. Man erhält 43 g 2-Methoxy-4-[4-methyl-5-ethyl-1,2,3-triazolyl(2)]-benzylchlorid vom Schemlzpunkt: 108 bis 110°C.

Beispiel 6

102 Teile einer Polyvinylchloridmasse, bestehend aus 70 Teilen Polyvinylchlorid, 30 Teilen eines Weichmachers, z.B. Dioctylphthalat, und 2 Teilen eines Stabilisators werden mit 0,01 Teilen des in Beispiel 1 genannten Weißtöners ca. 5 Minuten bei 150°C auf dem Zweiwalzenstuhl gewalzt und zu Folien ausgezogen. Zur Herstellung von gedeckten Folien werden der Mase vor dem Walzen 2,5 Teile Titandioxid zugesetzt. Man erhalt auf diese Weise vorzüglich weißgetönte PVC-Weichfolien.

**Patentansprüche**

1. Stilbenverbindungen der Formel

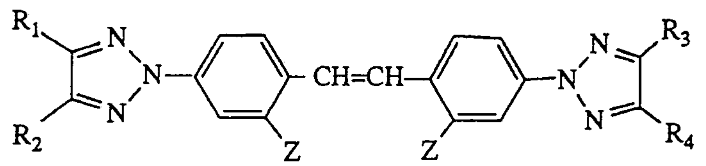

(I)

worin

$R_1$—$R_4$ unabhängig voneinander Wasserstoff oder $C_1$—$C_4$-Alkyl und

Z $C_1$—$C_4$-Alkoxy bedeuten.

2. Stilbenverbindungen gemäß Anspruch 1, dadurch gekennzeichnet, daß

$R_1$ Wasserstoff oder $C_1$—$C_4$-Alkyl,

$R_2$ $C_1$—$C_4$-Alkyl und

Z Methoxy bedeuten.

3. Stilbenverbindungen gemäß Anspruch 1 der Formel

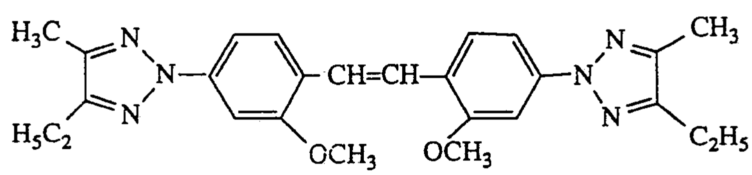

**Revendications**

1. Composés de stilbène de formule

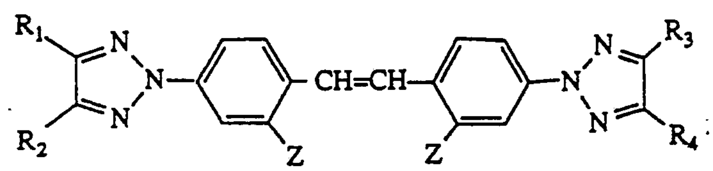

(I)

dans laquelle

$R_1$ à $R_4$ représentent, indépendamment les uns des autres, l'hydrogène ou un groupe alkyle en $C_1$ à $C_4$ et

Z est un groupe alkoxy en $C_1$ à $C_4$.

2. Composés de stilbène suivant la revendication 1, caractérisés en ce que

$R_1$ est l'hydrogène ou un groupe alkyle en $C_1$ à $C_4$,

$R_2$ est un groupe alkyle en $C_1$ à $C_4$ et

Z est un groupe méthoxy.

3. Composés de stilbène suivant la revendication 1, de formule

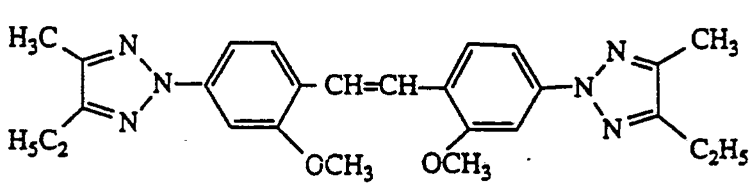

**Claims**

1. Stilbene compounds of the formula

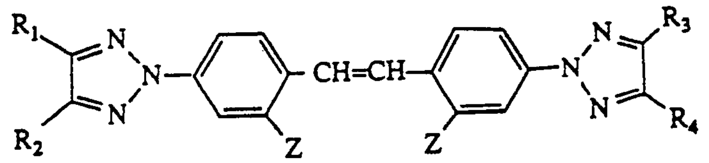

(I)

wherein

$R_1$—$R_4$ independently of one another denote hydrogen or $C_1$—$C_4$-alkyl and

Z denotes $C_1$—$C_4$-alkoxy.

2. Stilbene compounds according to Claim 1, characterized in that

$R_1$ denotes hydrogen or $C_1$—$C_4$-alkyl,

$R_2$ denotes $C_1$—$C_4$ alkyl and

Z denotes methoxy.

3. Stilbene compound according to Claim 1, of the formula